# EUROPÄISCHE PATENTSCHRIFT

(11) **EP 3 470 471 B1**
(45) Veröffentlichungstag und Bekanntmachung des Hinweises auf die Patenterteilung: **07.12.2022**
(21) Anmeldenummer: 18197061.7
(22) Anmeldetag: 27.09.2018
(51) Int. Cl.: C07D 209/32, C08K 5/3417, C08L 77/02, C08L 77/06, C09B 23/01, D01F 1/04, D01F 6/60, C09B 23/10, C08K 5/00

(54) **METHINFARBSTOFFE**
METHINE DYES
COLORANTS MÉTHYNE

(30) Priorität: 13.10.2017 EP 17196373
(43) Veröffentlichungstag der Anmeldung: 17.04.2019
(73) Patentinhaber: LANXESS Deutschland GmbH, 50569 Köln (DE)
(72) Erfinder: BORST, Hans-Ulrich, 50189 Elsdorf (DE); LINKE, Frank, 51069 Köln (DE); MICHAELIS, Stephan, 51519 Odenthal (DE)

(56) Entgegenhaltungen:
- EP-A1- 3 048 138
- EP-A2- 0 276 016
- DE-A1- 3 222 024
- DE-A1- 10 117 464
- ALHAMA ARJONA-ESTEBAN ET AL: "Influence of Solid-State Packing of Dipolar Merocyanine Dyes on Transistor and Solar Cell Performances", JOURNAL OF THE AMERICAN CHEMICAL SOCIETY, Bd. 137, Nr. 42, 15. Oktober 2015 (2015-10-15), Seiten 13524-13534, XP055454205, US ISSN: 0002-7863, DOI: 10.1021/jacs.5b06722

## Beschreibung

Die vorliegende Erfindung betrifft neue Methinfarbstoffe, Verfahren zu deren Herstellung und deren Verwendung zum Färben von Kunststoffen.

Obwohl es auf dem Markt bereits eine Vielzahl gelber Farbmittel zur Kunststoffeinfärbung gibt, besteht dennoch Bedarf an neuen Farbmitteln mit verbesserten Eigenschaften. Insbesondere besteht ein Verbesserungsbedarf der bekannten Farbmittel hinsichtlich ihrer Echtheiten. Dies gilt insbesondere für die Verwendung zum Massefärbung von Polyamid.

Das Massefärben von synthetischen Polyamiden stellt an die verwendeten Farbmittel höhere Anforderungen als das Massefärben anderer Kunststoffe. Die Verarbeitungstemperaturen der synthetischen Polyamide, insbesondere in Kombination mit Glasfasern, liegen entscheidend höher und auch die chemische Reaktivität der geschmolzenen Polyamide, insbesondere des Polyamids 6,6, ist wesentlich grösser, so dass die Hitzestabilität der verwendeten Farbmittel außerordentlich gut sein muss. Generell besitzen Pigmente eine hohe Thermostabilität. Es gibt jedoch wenige Pigmente, welche den hohen Anforderungen beim Massefärben von Kunststoffen genügen, insbesondere wenn zusätzlich auch eine hohe Lichtbeständigkeit verlangt wird.

Aus dem Stand der Technik sind Pigmente bekannt, die für die Färbung von Kunststoffen in gelben Farbtönen geeignet sind.

In der DE-A 3543512 A1 werden Pigmente auf Basis von Azofarblacken (Bayplast^{®} Gelb G) beschrieben, die zur Einfärbung von Polyamid in gelben Farbtönen verwendet werden können.

In der EP-A 0074515 werden Pigmente auf Basis von Nickelazobarbitursäurekomplexen offenbart, die ebenfalls zur Erzielung gelber Färbungen von Polyamid eingesetzt werden können.

Weiterhin lange bekannt ist die Verwendung von Pigment Yellow 192 (C.I. 507300) zum Erzielen von gelben Kunststoffeinfärbungen.

Die genannten Pigmente besitzen zwar eine gute Thermostabilität, allerdings können damit keine transparenten Färbungen von Kunststoffen erzielt werden. Außerdem können Pigmente die mechanischen Eigenschaften der Polymere verschlechtern. Aus dem Stand der Technik ist bekannt, lösliche Farbstoffe (Solvent Dyes) einzusetzen um Kunststoffe transparent in gelben Farbtönen einzufärben. Die mechanischen Eigenschaften der Polymere werden durch Farbstoffe in der Regel nicht negativ beeinflusst.

Bekannte lösliche Gelbfarbstoffe sind z.B. Solvent Yellow 114 (C.I. 47020) aus der Klasse der Chinophthalonfarbstoffe, Solvent Yellow 160:1 (C.I. 55165) aus der Klasse der Cumarinfarbstoffe sowie Solvent Yellow 179 (N-2-((4-Cyclohexyl)phenoxy)ethyl-N-ethyl-4-(2,2-dicyanoethenyl)-3-methylaniline) und Solvent Yellow 93 (C.I. 48160), beide aus der Klasse der Methinfarbstoffe.

Die Eigenschaften dieser, aus dem Stand der Technik bekannten gelben Farbmittel sind jedoch nicht immer ausreichend für die mittlerweile bestehenden technischen Anforderungen und sind insbesondere hinsichtlich ihrer Echtheiten, insbesondere ihrer Thermostabilität, noch verbesserungsbedürftig.

Weiterhin sind aus der EP-A 3 048 138 gelbe Methinfarbstoffe mit guten Lichtechtheiten bekannt, die auch hinsichtlich ihrer Thermostabilität gegenüber dem oben dargestellten Stand der Technik eine Verbesserung darstellen, aber trotzdem noch weiter verbesserungswürdig sind, da die anwendungstechnischen Anforderungen bei der Polyamid-Einfärbung noch weiter gestiegen sind.

Aus der EP-A 0 276 016 sind photopolymerisierbare Zusammensetzungen bekannt, die Farbmittel auf Basis von Xanthenen, Cumarinen oder Merocyaninfarbstoffen enthalten. Gegenstand der Anmeldung sind Methinfarbstoffe der Formel (I) 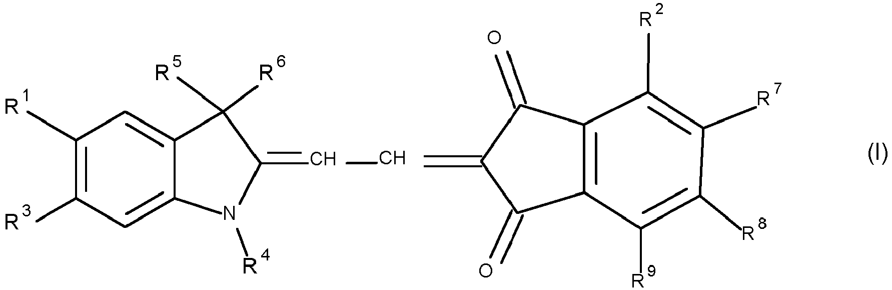 worin
- R¹: für Wasserstoff, Halogen, COOH oder COOR¹⁰ steht,
- R²: für Wasserstoff, Halogen Alkyl oder Alkoxy steht,
- R³: für Wasserstoff, Halogen, COOH, COOR¹¹ oder CN steht,
- R⁴: für Alkyl oder Phenyl steht,
- R⁵ und R⁶: unabhängig voneinander für Alkyl stehen,
- R⁷: für Wasserstoff, Halogen, Alkyl oder Alkoxy steht,
- R⁸: für Wasserstoff, Halogen, Alkyl oder Alkoxy steht,
- R⁹: für Wasserstoff, Halogen, Alkyl oder Alkoxy steht, und
- R¹⁰ und R¹¹: unabhängig voneinander für Alkyl stehen.

Die vorliegende Erfindung betrifft Methinfarbstoffe der Formel (I), worin
- R¹: für Wasserstoff, Halogen, COOH oder COOR¹⁰ steht,
- R²: für Wasserstoff, Halogen Alkyl oder Alkoxy steht,
- R³: für Wasserstoff, Halogen, COOH, COOR¹¹ oder CN steht,
- R⁴: für Alkyl oder Phenyl steht,
- R⁵ und R⁶: unabhängig voneinander für Alkyl stehen,
- R⁷: für Wasserstoff, Halogen, Alkyl oder Alkoxy steht,
- R⁸: für Wasserstoff, Halogen, Alkyl oder Alkoxy steht,
- R⁹: für Wasserstoff, Halogen, Alkyl oder Alkoxy steht, und
- R¹⁰ und R¹¹: unabhängig voneinander für Alkyl stehen,
mit der Bedingung, dass R¹ und R³ nicht gleichzeitig für Wasserstoff stehen.

Alkyl in den Bedeutungen von R⁴ bis R¹¹ bezeichnet beispielsweise geradkettiges oder verzweigtes C₁-C₆-Alkyl, vorzugsweise geradkettiges oder verzweigtes C₁-C₄-Alkyl, insbesondere Methyl, Ethyl, n- und iso-Propyl sowie n-, iso- und tert.-butyl, das jeweils gegebenenfalls ein- oder mehrfach, gleich oder verschieden substituiert sein kann, beispielsweise durch Halogen, wie Chlor, Brom oder Fluor, sowie durch -OH, -CN, -NH₂ oder C₁-C₆-Alkoxy.

Alkoxy in den Bedeutungen von R² und R⁷ bis R⁹ bezeichnet beispielsweise geradkettiges oder verzweigtes C₁-C₆-Alkoxy, vorzugsweise geradkettiges oder verzweigtes C₁-C₄-Alkoxy, insbesondere Methoxy, Ethoxy, n- und iso-Propoxy sowie n-, iso- und tert.-Butoxy.

Halogen in den Bedeutungen von R³ und R⁷ bis R¹¹ bezeichnet beispielweise Fluor, Chlor oder Brom.

In einerbevorzugten alternativen Ausführungsform sind Farbstoffe der Formel (I) bevorzugt, worin
- R¹: für Wasserstoff, Halogen, COOH oder COOR¹⁰ steht,
- R²: für Wasserstoff, Halogen, C₁-C₄-Alkyl, das gegebenenfalls ein- bis dreifach, gleich oder verschieden durch Halogen substituiert ist, oder für C₁-C₄-Alkoxy steht,
- R³: für Wasserstoff, Halogen, COOH, COOR¹¹ oder CN steht,
- R⁴: für C₁-C₄- Alkyl oder Phenyl steht,
- R⁵ und R⁶: jeweils unabhängig voneinander für C₁-C₄-Alkyl stehen,
- R⁷: für Wasserstoff, Halogen, C₁-C₄-Alkyl das gegebenenfalls ein- bis dreifach durch Halogen substituiert ist oder für C₁-C₄-Alkoxy steht,
- R⁸: für Wasserstoff, Halogen, C₁-C₄-Alkyl das gegebenenfalls ein- bis dreifach durch Halogen substituiert ist oder für C₁-C₄-Alkoxy steht,
- R⁹: für Wasserstoff, Halogen, C₁-C₄-Alkyl das gegebenenfalls ein- bis dreifach durch Halogen substituiert ist oder für C₁-C₄-Alkoxy steht und
- R¹⁰ und R¹¹: jeweils unabhängig voneinander für C₁-C₄-Alkyl stehen,
mit der Bedingung, dass R¹ und R³ nicht gleichzeitig für Wasserstoff stehen.

Besonders bevorzugt sind Farbstoffe der Formel (I), worin
- R¹: für Wasserstoff, Fluor, Chlor, COOH oder COOR¹⁰ steht,
- R²: für Wasserstoff, Fluor, Chlor, Methyl, Ethyl, Trifluormethyl, Methoxy oder Ethoxy steht,
- R³: für Wasserstoff, Fluor, Chlor, COOH, COOR¹¹ oder CN steht,
- R⁴: für Methyl, Ethyl oder Phenyl steht,
- R⁵ und R⁶: jeweils unabhängig voneinander für Methyl oder Ethyl stehen,
- R⁷: für Wasserstoff, Fluor, Chlor, Methyl, Ethyl, Trifluormethyl, Methoxy oder Ethoxy steht,
- R⁸: für Wasserstoff, Fluor, Chlor, Methyl, Ethyl, Trifluormethyl, Methoxy oder Ethoxy steht,
- R⁹: für Wasserstoff, Fluor, Chlor, Methyl, Ethyl, Trifluormethyl, Methoxy oder Ethoxy steht und
- R¹⁰ und R¹¹: jeweils unabhängig voneinander für Methyl oder Ethyl stehen,
mit der Bedingung, dass R¹ und R³ nicht gleichzeitig für Wasserstoff stehen.

Ganz besonders bevorzugt sind Farbstoffe der Formel (I), worin
- R¹: für Chlor oder COOR¹⁰ steht,
- R²: für Wasserstoff, Fluor, Chlor, CF₃, Methyl oder Methoxy steht,
- R³: für Wasserstoff steht,
- R⁴: für Methyl steht
- R⁵ und R⁶: für Methyl stehen
- R⁷: für Wasserstoff, Fluor, Chlor, Methyl oder Methoxy steht,
- R⁸: für Wasserstoff, Fluor, Chlor, Methyl oder Methoxy steht,
- R⁹: für Wasserstoff, Fluor, Chlor, Methyl oder Methoxy steht und
- R¹⁰: für Methyl steht.

Die Farbstoffe der Formel (I) können als Stereoisomere vorliegen. Die Formel (I) umfasst insbesondere die folgenden vier E- und Z-Isomere der Formeln (la) bis (Id): 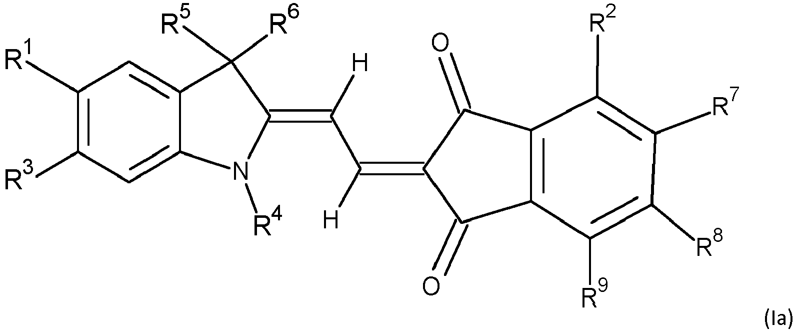 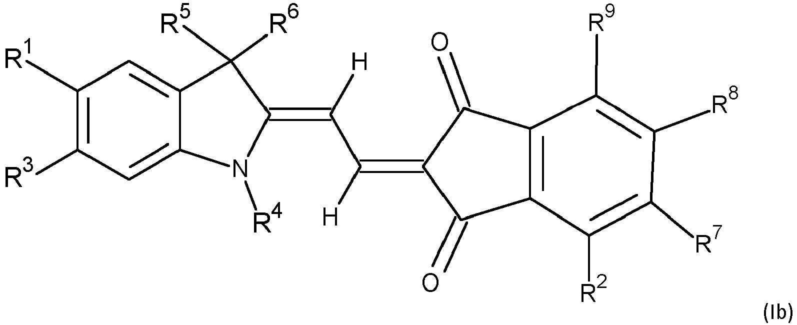 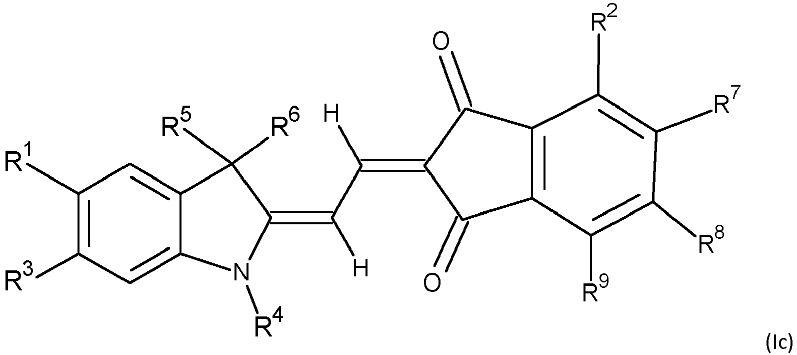 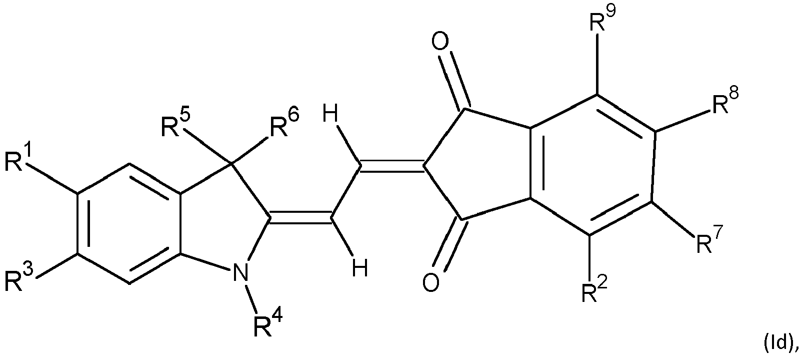 worin die Substituenten R¹ bis R⁹ die für Formel (I) angegebenen allgemeinen und bevorzugten Bedeutungen haben.

In einer weiteren alternativen Ausführungsform betrifft die vorliegende Erfindung Methinfarbstoffe der Formel (la), worin die Substituenten R¹ bis R⁹ die für Formel (I) angegebenen allgemeinen und bevorzugten Bedeutungen haben.

Mit den erfindungsgemäßen Farbstoffen der Formel (I) lassen sich gelbe bis orange Einfärbungen von Kunststoffen, insbesondere von Polyamiden, erzielen, die sich durch verbesserte Lichtechtheiten und verbesserte Thermostabilität gegenüber den bekannten, für diese Zwecke eingesetzten, gelben Farbstoffen auszeichnen. Darüber hinaus besitzen die erfindungsgemäßen Farbstoffe gegenüber den bekannten Farbstoffen überraschender Weise auch eine verbesserte Farbstärke.

Mit den erfindungsgemäßen Farbstoffen gelingt es, die bisher realisierten Eigenschaftsprofile bekannter gelber Farbstoffe für Kunststoffeinfärbungen deutlich zu übertreffen. Weiterer Gegenstand der vorliegenden Erfindung ist die Verwendung der erfindungsgemäßen Farbstoffe der Formel (I) zum Massefärben von Kunststoffen. Dabei können die erfindungsgemäßen Farbstoffe einzeln oder in beliebiger Mischung untereinander eingesetzt werden.

Unter Massefärben werden hierbei insbesondere Verfahren verstanden, bei denen der Farbstoff in die geschmolzene Kunststoffmasse eingearbeitet wird, z.B. unter Zuhilfenahme eines Extruders, oder bei denen der Farbstoff bereits den Ausgangskomponenten zur Herstellung des Kunststoffes, z.B. Monomeren vor der Polymerisation, zugesetzt wird.

Besonders bevorzugte Kunststoffe sind Thermoplaste, beispielsweise Vinylpolymere, Polyester, Polyamide sowie Polyolefine, insbesondere Polyethylen und Polypropylen, Polycarbonate und Polyamid. Ganz besonders bevorzugt sind Polyamide, insbesondere Polyamid 6.6 und Polyamid 6.

Die Bezeichnung Polyamide wird im Sinne der vorliegenden Erfindung als Bezeichnung für synthetische, technisch verwendbare thermoplastische Kunststoffe verwendet und grenzt diese Stoffklasse damit von den chemisch verwandten Proteinen ab. Fast alle bedeutsamen Polyamide leiten sich von primären Aminen ab, denn die sich wiederholende Einheit besteht aus der funktionellen Gruppe -CO-NH-. Daneben existieren auch Polyamide von sekundären Aminen (-CO-NR-, R = organischer Rest). Als Monomere für die Herstellung der Polyamide dienen insbesondere Aminocarbonsäuren, Lactame und/oder Diamine und Dicarbonsäuren.

Polyamid 6.6 wird üblicher Weise aus Hexamethylendiamin (HMD) und Adipinsäure hergestellt. Es entsteht durch eine Polykondensation unter Wasserabspaltung.

Polyamid 6 ist erhältlich durch Ringöffnungspolymerisation von ε-Caprolactam mit Wasser als Starter.

Geeignete Vinylpolymere sind Polystyrol, Styrol-Acrylnitril-Copolymere, Styrol-Butadien-Copolymere, Styrol-Butadien-Acrylnitril-Terpolymere, Polymethacrylat, Polyvinylchlorid u.a.

Geeignete Polyester sind beispielsweise Polyethylenterephthalate, Polycarbonate und Celluloseester.

Die zu färbenden Kunststoffe können einzeln oder in Gemischen untereinander, als plastische Massen oder Schmelzen vorliegen.

Bei ihrem Einsatz zum Massefärben von Kunststoffen werden die erfindungsgemäßen Farbstoffe (I) vorzugsweise in feinverteilter Form zur Anwendung gebracht, wobei Dispergiermittel mitverwendet werden können aber nicht müssen.

Bei ihrem Einsatz zum Massefärben von Kunststoffen können die erfindungsgemäßen Farbstoffe (I) beispielsweise direkt beim Prozess der Kunststoffherstellung nach der erfolgten Polymerisation eingesetzt werden. Dabei wird vorzugsweise mindestens ein erfindungsgemäßer Farbstoff (I) mit dem Kunststoffgranulat trocken vermischt oder vermahlen und dieses Gemisch beispielsweise auf Mischwalzen oder in Schnecken plastifiziert und homogenisiert. Man kann die erfindungsgemäßen Farbstoffe (I) aber auch der schmelzflüssigen Masse zugeben und durch Rühren homogen verteilen. Das derart vorgefärbte Material kann dann wie üblich z.B. durch Verspinnen zu Borsten, Fäden usw. oder durch Extrusion oder im Spritzguss-Verfahren zu Formteilen weiterverarbeitet werden.

Da die Farbstoffe (I) gegenüber Polymerisationskatalysatoren, insbesondere Peroxiden, beständig sind, ist es auch möglich, die erfindungsgemäßen Farbstoffe (I) den monomeren Ausgangsmaterialien für die Kunststoffherstellung, z. B. von Polymethylmethacrylat (PMMA) zuzusetzen und dann in Gegenwart von Polymerisationskatalysatoren zu polymerisieren. Dazu wird der Farbstoff vorzugsweise in den monomeren Komponenten gelöst oder mit ihnen innig vermischt.

Die erfindungsgemäßen Farbstoffe der Formel (I) werden vorzugsweise zum Färben der genannten Kunststoffe, insbesondere Polyamid, in Mengen von 0,0001 bis 1 Gew.-%, insbesondere 0,01 bis 0,5 Gew.-%, bezogen auf die Menge an Polymer eingesetzt.

Durch Zusatz von in den Polymeren unlöslichen Pigmenten, wie z.B. Titandioxid, können entsprechende wertvolle gedeckte Färbungen erhalten werden.

Titandioxid kann in einer Menge von 0,01 bis 10 Gew.-%, vorzugsweise 0,1 bis 5 Gew.-%, bezogen auf die Polymermenge, verwendet werden.

Gegenstand der vorliegenden Erfindung ist weiterhin ein Verfahren zum Massefärben von Kunststoffen, wobei mindestens ein Farbstoff der Formel (I) mit mindestens einem Kunststoff, vorzugsweise in Granulatform, trocken vermischt oder vermahlen wird und dieses Gemisch z.B. auf Mischwalzen oder in Schnecken plastifiziert und homogenisiert wird.

Man kann die erfindungsgemäßen Farbstoffe (I) aber auch der schmelzflüssigen Masse zugeben und durch Rühren homogen verteilen. Ebenfalls ist es möglich, die erfindungsgemäßen Farbstoffe (I) bei der Kunststoffherstellung den monomeren Ausgangskomponenten zuzusetzen und anschließend zu polymerisieren.

Das derart vorgefärbte Material kann dann wie üblich z.B. durch Verspinnen zu Borsten, Fäden usw. oder durch Extrusion oder im Spritzguss-Verfahren zu Formteilen weiterverarbeitet werden.

Nach dem erfindungsgemäßen Verfahren erhält man transparente bzw. gedeckte brillante gelbe Färbungen mit sehr guter Hitze- und Lichtbeständigkeit.

Zur Durchführung des erfindungsgemäßen Verfahrens können auch Mischungen der erfindungsgemäßen Farbstoffe der Formel (I) mit anderen Farbstoffen und/oder anorganischen und/oder organischen Pigmenten eingesetzt werden.

Weiterer Gegenstand der vorliegenden Erfindung ist ein Verfahren zur Herstellung der erfindungsgemäßen Farbstoffe der Formel (I).

Die erfindungsgemäßen Farbstoffe der Formel (I) können hergestellt werden, indem man mindestens einen Aldehyd der Formel (II) 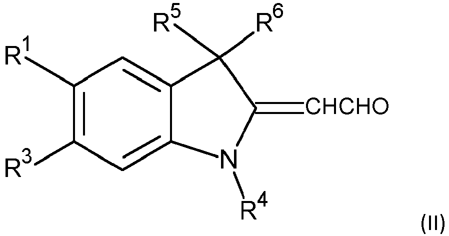 worin
R¹, R³, R⁴, R⁵ und R⁶ die für Formel (I) angegebenen allgemeinen und bevorzugten Bedeutungen haben,
mit mindestens einem Indan-1,3-dion -Derivat der Formel (III) 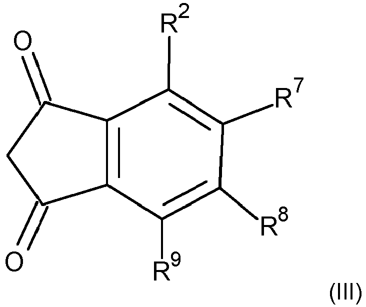
worin
   R², R⁷, R⁸ und R⁹ die für Formel (I) angegebenen allgemeinen und bevorzugten Bedeutungen haben,
   umsetzt.

Die Aldehyd der Formel (II) können als Stereoisomere vorliegen. Die Formel (II) umfasst die beiden möglichen E- und Z-Formen.

Das Verfahren zur Herstellung der erfindungsgemäßen Farbstoffe (I) durch Umsetzung der Aldehyde der Formel (II) mit den Indan-1,3-dion -Derivaten der Formel (III) kann in an sich bekannter Art und Weise durchgeführt werden.

Das Verfahren zur Herstellung der erfindungsgemäßen Farbstoffe (I) wird im Allgemeinen bei einer Temperatur im Bereich von -10 bis 180°C, bevorzugt von 0 bis 100°C und besonders bevorzugt von 10 bis 90°C durchgeführt.

Das Verfahren zur Herstellung der erfindungsgemäßen Farbstoffe (I) wird im Allgemeinen bei einem Druck von 900 bis 1100 hPa, vorzugsweise bei Normaldruck durchgeführt.

Das Verfahren zur Herstellung der erfindungsgemäßen Farbstoffe (I) kann in Gegenwart von mindestens einem Lösungsmittel durchgeführt werden. Geeignete Lösungsmitte sind beispielsweise solche aus der Reihe der Alkohole und Formamide. Vorzugsweise wird das Verfahren zur Herstellung der erfindungsgemäßen Farbstoffe (I) in Gegenwart von mindestens einem Alkohol aus der Reihe Methanol, Ethanol, Propanol, und/oder mindestens einem Formamid aus der Reihe Dimethylformamid und Diethylformamid, besonders bevorzugt in Gegenwart von Methanol und/oder Dimethylformamid durchgeführt.

Das Verfahren zur Herstellung der erfindungsgemäßen Farbstoffe (I) wird in Gegenwart von mindestens einer Base durchgeführt. Geeignete Basen sind beispielsweise Alkalihydroxide und Alkalialkoholate. Bevorzugt ist die Verwendung von Lithiumhydroxid, Natriumhydroxid, Kaliumhydroxid und/oder Kalium-tert.-butylat, besonders bevorzugt von Natriumhydroxid und/oder Kalium-tert.-butylat.

Im Allgemeinen wird das Verfahren zur Herstellung der erfindungsgemäßen Farbstoffe (I) so durchgeführt, dass zunächst der Aldehyd (II) vorlegt und das Indan-1,3-dion -Derivat (III) hinzugefügt wird und nach erfolgter Umsetzung die Verbindungen der Formel (I) isoliert wird. Die Isolierung kann durch übliche Verfahren, vorzugsweise durch Filtration, erfolgen. Das erhaltene Reaktionsprodukt kann gegebenenfalls durch weitere Verfahrensschritte wie Waschen und Trocknen aufgearbeitet werden.

Zur Durchführung des Verfahrens wird im Allgemeinen pro Mol an Aldehyd (II) 0,8 bis 1,5 Mol an Indan-1,3-dion Derivat (III) eingesetzt. Bevorzugt wird pro Mol an Aldehyd (II) 0,9 bis 1,1 Mol an Indan-1,3-dion Derivat (III) eingesetzt und besonders bevorzugt wird pro Mol an Aldehyd (II) 1 Mol an Indan-1,3-dion Derivat (III) eingesetzt.

Indan-1,3-dion Derivate der Formel (III) sind bekannt und können beispielsweise als Handelsprodukte der Firma Alfa Acer bezogen werden.

Die Aldehyde der Formel (II) sind ebenfalls bekannt und können beispielsweise, in dem Fachmann bekannter Art und Weise, in einer zweistufigen Synthese hergestellt werden. Dabei wird in einer ersten Stufe a) mindestens ein Indol-Derivat der Formel (IV) 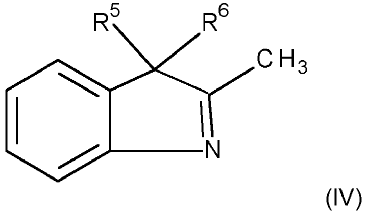 worin
R⁵ und R⁶ die für Formel (I) angegebenen allgemeinen und bevorzugten Bedeutungen haben,
mit mindestens einem Alkylierungsreagenz umgesetzt, und anschließend in einer zweiten Stufe b) das Zwischenprodukt der ersten Stufe mit mindestens einem Formylierungsreagenz umgesetzt.

Umsetzungen der in Stufe b) beschrieben Art sind ist in der Literatur unter dem Namen Vilsmeier-Reaktion bekannt.

Im Allgemeinen wird die Umsetzung in Stufe a) so durchgeführt, dass man das Indol-Derivat der allgemeinen Formel (IV) vorlegt und gegebenenfalls in Gegenwart eines Lösungsmittels das Alkylierungsmittel hinzufügt.

Die erste Stufe a) der Umsetzung wird im Allgemeinen bei einer Temperatur im Bereich zwischen 10 und 80°C bevorzugt von 20 bis 70°C und besonders bevorzugt von 30 bis 60°C durchgeführt.

Die Umsetzung in Stufe a) wird im Allgemeinen bei einem Druck von 900 bis 1100 hPa, vorzugsweise bei Normaldruck durchgeführt.

Die Umsetzung in Stufe a) kann in Gegenwart von mindestens einem Lösungsmittel durchgeführt werden. Geeignete Lösungsmitte sind beispielsweise solche aus der Reihe der Alkohole und Wasser. Vorzugsweise wird die Umsetzung in Stufe a) in Gegenwart von Wasser als Lösungsmittel durchgeführt.

Als Alkylierungsreagenz geeignet sind prinzipiell alle bekannten Alkylierungsreagenzien (siehe z. B. K. Schwetlick, Organikum, VEB Deutscher Verlag der Wissenschaften, Berlin, 15. Auflage 1977, Seite 260, 253, 674), wie beispielsweise Dimethylsulfat, Methyliodid oder Diazomethan. Bevorzugt ist die Verwendung von Dimethylsulfat.

Im allgemeinen wird pro Mol an Indol-Derivat mindestens ein Mol an Alkylierungsreagenz eingesetzt. Abhängig von der Struktur des Indol-Derivats kommen, entsprechend der vorgegebenen Stöchiometrie, auch höhere Molmengen zum Einsatz. Vorzugsweise wird pro Mol an Indol-Derivat (IV) 0,9 bis 1,1 Mol, besonders bevorzugt 1 Mol an Alkylierungsreagenz eingesetzt.

Das in Stufe a) hergestellte Zwischenprodukt kann durch übliche Verfahren, beispielsweise durch Filtration isoliert werden. Vorzugsweise wird das in Stufe a) hergestellte Zwischenprodukt ohne Isolierung direkt weiter in der anschließenden Stufe b) umgesetzt.

Im Allgemeinen wird die Umsetzung in Stufe b) so durchgeführt, dass man die alkylierte Verbindung aus der ersten Stufe a) in Form der erhaltenen Reaktionslösung vorlegt und das Formylierungsreagenz, gegebenenfalls in Gegenwart mindestens eines Lösungsmittels, hinzufügt und anschließend den so hergestellten Aldehyd der Formel (II), gegebenenfalls durch Zugabe einer geeigneten Menge eines geeigneten Fällungsmittels ausfällt, und anschließen den Aldehyd der Formel (II) durch übliche Verfahren, beispielsweise durch Filtration, isoliert.

Die Umsetzung in Stufe b) wird im Allgemeinen bei einer Temperatur im Bereich zwischen 10 und 80°C bevorzugt von 20 bis 70°C und besonders bevorzugt von 30 bis 60°C durchgeführt.

Die Umsetzung in Stufe b) wird im Allgemeinen bei einem Druck 900 bis 1100 hPa, vorzugsweise bei Normaldruck durchgeführt.

Die Umsetzung in Stufe b) kann in Gegenwart von mindestens einem Lösungsmittel durchgeführt werden. Geeignete Lösungsmitte sind beispielsweise Formamide. Bevorzugt sind Dimethylformamid, und Diethylformamid, besonders bevorzugt ist die Verwendung von Dimethylformamid. Bei Verwendung von Dimethylformamid ist es insbesondere bevorzugt dieses im Überschusses einzusetzen, wobei das Dimethylformamid dann zugleich als Formylierungsreagenz und als Lösungsmittel dient.

Als Formylierungsreagenz wird in Stufe b) im Allgemeinen eine Mischung aus mindestens einem Formamid und mindestens einem Phosphorsäurechlorid eingesetzt.

Bevorzugte Formamide sind Dimethylformamid, Diethylformamid und Dibutylformamid.

Bevorzugtes Phosphorsäurechlorid ist Phosphoroxychlorid.

Besonders bevorzugt wird als Formylierungsreagenz eine Mischung aus Dimethylformamid und Phosphoroxychlorid eingesetzt.

Im Allgemeinen wird pro Mol an alkylierter Verbindung aus Stufe 1 mindestens ein Mol an Formylierungsreagenz eingesetzt, bevorzugt 1,1 bis 1,5 Mol und besonders bevorzugt 1,1 bis 1 Mol.

Geeignete Fällungsmittel sind beispielsweise Alkohole wie Methanol und/oder Ethanol.

Bevorzugt wird als Fällungsmittel Methanol und/oder Ethanol, insbesondere Methanol eingesetzt.

Die Indol-Derivate der Formel (IV) sind dem Fachmann bekannt. Sie können in an sich bekannter Art und Weise in einer zweistufigen Synthese hergestellt werden durch Umsetzung eines Anilin-Derivats der Formel (V) 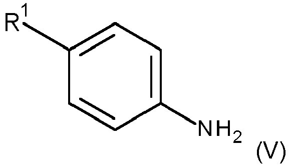 worin
R¹ die für Formel (I) angegebene allgemeine und bevorzugte Bedeutung hat,
mit einem Diazotierungsreagenz und anschließender Umsetzung unter Ringschluss mit einem Keton der Formel (VI) 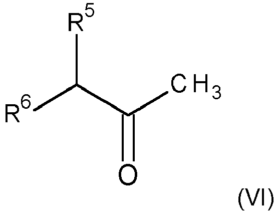
worin
   R⁵ und R⁶ die für Formel (I) angegebene allgemeine und bevorzugte Bedeutung haben.

Die Diazotierungsreaktion wird im allgemeinen durchgeführt, indem man das Anilin-Derivat vorlegt und das Diazotierungsreagenz bei einer Temperatur im Bereich zwischen 0 bis 10°C , bei Normaldruck in einem wässerigen Medium hinzufügt.

Als Diazotierungsreagenz kommt prinzipiell jedes geeignete Diazotierungsreagenz in Frage. Bevorzugt ist die Verwendung einer wässerigen Natriumnitrit-Lösung.

Im Allgemeinen wird das Diazotierungsreagenz in einer Menge von mindestens zwei Mol bezogen auf das Anilin-Derivat (V) eingesetzt.

Die Ringschluss-Reaktion mit dem Keton der Formel (VI) wird in an sich bekannter Art und Weise einer Eintopf-Reaktion durchgeführt, indem man das Diazoniumsalz des Anilin-Derivats (V) zum Hydrazon reduziert und das Hydrazon mit dem Keton der allgemeinen Formel (VI) umsetzt, bevorzugt bei einer Temperatur im Bereich von 40 bis 100°C, bevorzugt in wässriger Lösung, und anschließend das Indol-Derivat der Formel (IV) durch übliche Verfahren, vorzugsweise Filtration, isoliert und wäscht.

Die Anilin-Derivate der Formel (V) sowie die Ketone der Formel (VI) sind bekannt und können als Handelsprodukte bezogen werden, beispielsweise von den Firmen Alfa Acer oder Sigma-Aldrich.

Die Erfindung wird erläutert, jedoch nicht beschränkt durch die folgenden Beispiele, in denen die Teile sich auf das Gewicht beziehen und Prozentangaben Gewichtsprozente (Gew.-%) bedeuten.

### Beispiel 1

Herstellung der erfindungsgemäßen Verbindung der Formel (I) 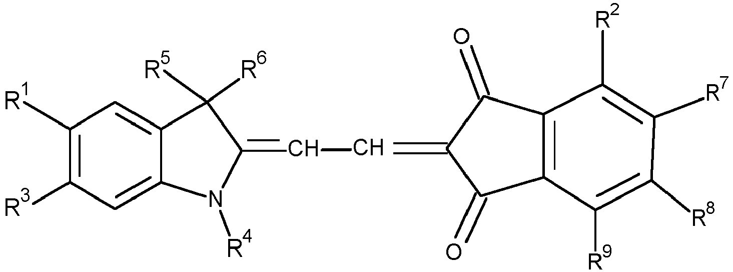 mit R¹ = COOCH₃; R² = H; R³ = H; R⁴, R⁵ und R⁶= -CH₃; R⁷, R⁸ und R⁹ = H

In 150 ml Essigsäureanhydrid wurden 25,9 g (= 0,1 Mol) Aldehyd der Formel (II) mit R¹ = COOCH₃; R³ = H; R⁴, R⁵ und R⁶ = CH₃ sowie 14,6 g (= 0,1 Mol) Indan-1,3-dion eingetragen. Anschließend wurde mit 2 g Ammoniumchlorid versetzt. Danach wurde die Mischung auf eine Temperatur von 105°C erwärmt und für 6 Stunden gerührt. Danach wurde auf 25°C abgekühlt und mit 100 ml Methanol versetzt. Das Reaktionsprodukt wurde auf einer Nutsche isoliert. Der Filterkuchen wurde mit ca. 100 ml Methanol und ca. 500 ml Wasser mit einer Temperatur von 90°C gewaschen. Das gewaschene Produkt wurde im Vakuumtrockenschrank bei einer Temperatur von 80°C und einem Druck von 200 hPa getrocknet.

Ausbeute: 30,9 g (entspricht 80% der Theorie), Schmelzpunkt 245°C

### Beispiele 2 bis 9

Herstellung von erfindungsgemäßen Verbindungen der Formel (I) worin die Substituenten R¹ bis R⁹ die in Tabelle 1 aufgeführten Bedeutungen haben.

**Tabelle 1**

| **Beispiel** | **R¹** | **R²** | **R³** | **R⁴** | **R⁵** | **R⁶** | **R⁷** | **R⁸** | **R⁹** |
|---|---|---|---|---|---|---|---|---|---|
| 2 | Cl | H | H | CH₃ | CH₃ | CH₃ | H | H | H |
| 3 | COOCH₃ | H | H | CH₃ | CH₃ | CH₃ | OCH₃ | OCH₃ | H |
| 4 | COOCH₃ | CH₃ | H | CH₃ | CH₃ | CH₃ | H | H | OCH₃ |
| 5 | COOCH₃ | Cl | H | CH₃ | CH₃ | CH₃ | H | H | H |
| 6 | COOCH₃ | H | H | CH₃ | CH₃ | CH₃ | CH₃ | H | H |
| 7 | COOCH₃ | Cl | H | CH₃ | CH₃ | CH₃ | Cl | Cl | Cl |
| 8 | COOCH₃ | H | H | CH₃ | CH₃ | CH₃ | Cl | Cl | H |
| 9 | COOCH₃ | H | H | CH₃ | CH₃ | CH₃ | OCH₃ | H | H |

Die Herstellung und Aufarbeitung der Verbindungen der Beispiele 2 bis 9 erfolgte jeweils analog zu Beispiel 1, jedoch mit folgenden Abweichungen:

### Beispiel 2

Anstelle des in Beispiel 1 verwendeten Aldehyds wurden 23,5 g (0,10 Mol) Aldehyd der Formel (II) mit R¹ = Cl; R³ = H und R⁴, R⁵ und R⁶ = CH₃ eingesetzt.

Ausbeute: 27,6 g (entspricht 76 % der Theorie), Schmelzpunkt 278°C.

### Beispiel 3

Anstelle des in Beispiel 1 verwendeten Indan-1,3-dion wurden 20,6 g (0,1 Mol) Indan-1,3-dion Derivat der Formel (III) mit R² und R⁹ = H und R⁷ und R⁸ = OCH₃ eingesetzt.

Ausbeute: 35,4 g (entspricht 79 % der Theorie), Schmelzpunkt 282°C.

### Beispiel 4

Anstelle des in Beispiel 1 verwendeten Indan-1,3-dion wurden 19,0 g (0,1 Mol) Indan-1,3-dion Derivat der Formel (III) mit R² = CH₃; R⁹ = OCH₃ und R⁷ und R⁸ = H eingesetzt.

Ausbeute: 32,4 g (entspricht 75 % der Theorie), Schmelzpunkt 281°C.

### Beispiel 5

Anstelle des in Beispiel 1 verwendeten Indan-1,3-dion wurden 18,1 g (0,10 Mol) Indan-1,3-dion Derivat der Formel (III) mit R² = Cl und R⁷, R⁸ und R⁹ = H eingesetzt.

Ausbeute: 32,9 g (entspricht 78 % der Theorie), Schmelzpunkt 284°C

### Beispiel 6

Anstelle des in Beispiel 1 verwendeten Indan-1,3-dion wurden 16,0 g (0,10 Mol) Indan-1,3-dion Derivat der Formel (III) mit R⁷ = CH₃ und R², R⁸ und R⁹ = H eingesetzt.

Ausbeute: 29,7 g (entspricht 74 % der Theorie), Schmelzpunkt 278°C

### Beispiel 7

Anstelle des in Beispiel 1 verwendeten Indan-1,3-dion wurden 28,4 g (0,10 Mol) Indan-1,3-dion Derivat der Formel (III) mit R², R⁷, R⁸ und R⁹ = Cl eingesetzt.

Ausbeute: 43,1 g (entspricht 82 % der Theorie), Schmelzpunkt 289°C

### Beispiel 8

Anstelle des in Beispiel 1 verwendeten Indan-1,3-dion wurden 21,5 g (0,10 Mol) Indan-1,3-dion Derivat der Formel (III) mit R² und R⁹ = H und R⁷ und R⁸ = Cl eingesetzt.

Ausbeute: 37,0 g (entspricht 81 % der Theorie), Schmelzpunkt 283°C

### Beispiel 9

Anstelle des in Beispiel 1 verwendeten Indan-1,3-dion wurden 17,6 g (0,10 Mol) Indan-1,3-dion Derivat der Formel (III) mit R², R⁸ und R⁹ = H und R⁷ = OCH₃ eingesetzt.

Ausbeute: 32,6 g (entspricht 78 % der Theorie), Schmelzpunkt 277°C

### Herstellung der Vorstufen

### Beispiel 10

Herstellung eines Aldehyds der Formel (II) 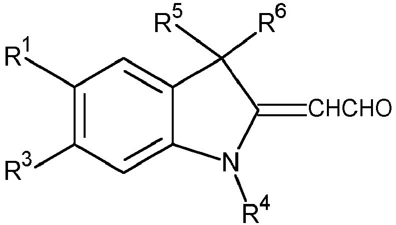 mit R¹ = COOCH₃; R³ = H und R⁴, R⁵ und R⁶ = CH₃
a) Diazotierung:
   In 270 g 30%ige Salzsäure wurden 139,9 g p-Aminobenzoesäure eingetragen und durch Kühlung von außen auf 0°C abgekühlt. Anschließend wurde mit 174 g einer 40%igen wässrigen Natriumnitrit Lösung versetzt. Es wurde für 30 Minuten gerührt und dann mit ca. 0,5 g Amidosulfonsäure der Nirit-Überschuss entfernt.
b) Herstellung des Hydrazons und Ringschluss:
   Eine Mischung aus 250 g Wasser und 660 g Natriumhydrogensulfit, in Form einer 39%igen wässrigen Lösung, wurde mit 80 g einer 40%igen wässrigen Natriumhydroxid-Lösung auf einen pH-Wert von 6,5 eingestellt. Im Laufe von ca. 30 Minuten wurde die in Schritt a) hergestellte Diazotierungslösung zugegeben, wobei durch Zugabe von 100 g einer 40%igen wässrigen Natriumhydroxid-Lösung der pH-Wert bei ca. 6,5 gehalten wurde. Anschließend wurde die Reaktionsmischung bei einer Temperatur von 40°C für ca. 1 Stunde gerührt. Danach wurden 560 g 96%ige Schwefelsäure und anschließend 86,1 g Methylisopropylketon zugetropft. Die Reaktionsmischung wurde auf 70°C erwärmt und für ca. 4 Stunden gerührt. Anschließend wurde die Reaktionsmischung auf 80°C erwärmt und nochmals für ca. 4 Stunden gerührt. Danach wurde die Reaktionsmischung auf 25°C abgekühlt und mit ca. 800 g einer 40%igen wässrigen Natriumhydroxid-Lösung auf einen pH-Wert von 6,5 eingestellt. Die Reaktionsmischung wurde für 30 Minuten gerührt und anschließend das Reaktionsprodukt auf einer Nutsche isoliert und mit 2 Liter Wasser gewaschen.
c) Herstellung des Aldehyds:
   Der wasserfeuchte Presskuchen des Ringschlussproduktes aus Stufe b) wurde in 1200 g Wasser eingetragen. Anschließend wurde mit ca. 70 g einer 40%igen wässrigen Natriumhydroxid-Lösung ein pH-Wert von 10 eingestellt. Im Laufe von 1 Stunde wurden 325 g Dimethylsulfat zugetropft und dabei durch Zugabe von 200 g einer 40%igen wässrigen Natriumhydroxid-Lösung der pH-Wert bei ca. 8,5 gehalten. Die Reaktionsmischung wurde auf 40°C erwärmt und für ca. 5 Stunden gerührt. Anschließend wurde die Reaktionsmischung auf 60°C erwärmt und nochmals für 1 Stunde gerührt. Die Reaktionsmischung wurde dann stehen gelassen wobei innerhalb von 1 Stude eine Phasentrennung erfolgte. Dann wurde die wässrige Phase abgetrennt. Im Vakuum bei 80°C und 20 hPa wurde restliches Wasser aus der organischen Phase entfernt. Danach wurden zu der organischen Phase 310 g Dimethylformamid zugetropft. Anschließend wurden bei 40°C 263 g Phosphoroxychlorid im Laufe von 3 Stunden zugegeben und die Reaktionsmischung für 5 Stunden gerührt. Anschließend wurde auf 20°C abgekühlt und mit 160 g Methanol versetzt. Dann wurde mit ca. 200 g einer 40%igen wässrigen Natriumhydroxid-Lösung ein pH-Wert von 11 eingestellt. Anschließend wurde die Reaktionsmischung für 60 Minuten gerührt und dann das Reaktionsprodukt auf einer Nutsche isoliert und mit 160 g Methanol und 2000 g Wasser gewaschen. Das gewaschene Produkt wurde im Vakuumtrockenschrank bei einer Temperatur von 80°C und einem Druck von 200 hPa getrocknet.

Ausbeute: 176,3 g (entspricht 68% der Theorie)

### Beispiele 11

Herstellung eines Aldehyds der Formel (II) 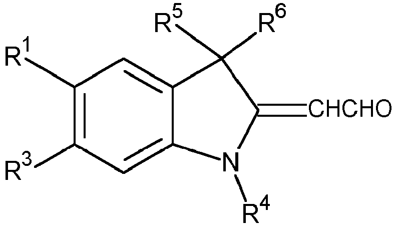 mit R¹ = COOCH₃; R³ = H und R⁴, R⁵ und R⁶ = CH₃
a) Diazotierung:
   Die Herstellung der Diazotierung erfolgte wie in Beispiel 10 a) angegeben, jedoch wurden anstelle von 270 g 30%iger Salzsäure und 139,9 g p-Aminobenzoesäure 268 g 30%ige Salzsäure und 127,6 g 4-Chloranilin verwendet.
b) Herstellung des Hydrazons:
   Die Herstellung des Hydrazons und der Ringschluss erfolgten analog zu Beispiel 10 b), es wurde jedoch die Diazotierungslösung aus Schritt 11 a) eingesetzt.
c) Herstellung des Aldehyds:
   Der wasserfeuchte Presskuchen des Ringschlussproduktes aus Stufe b) wurde in 1200 g Wasser eingetragen. Anschließend wurde mit ca. 5 g einer 40%igen wässrigen Natriumhydroxid-Lösung ein pH-Wert von 10 eingestellt. Im Laufe von 1 Stunde wurden 153 g Dimethylsulfat zugetropft und dabei durch Zugabe von 90 g einer 40%igen wässrigen Natriumhydroxid-Lösung der pH-Wert bei ca. 8,5 gehalten. Die Reaktionsmischung wurde auf 40°C erwärmt und für ca. 5 Stunden gerührt. Anschließend wurde die Reaktionsmischung auf 60°C erwärmt und nochmals für 1 Stunde gerührt. Die Reaktionsmischung wurde dann stehen gelassen wobei innerhalb von 1 Stunde eine Phasentrennung erfolgte. Dann wurde die wässrige Phase abgetrennt. Im Vakuum bei 80°C und 20 hPa wurde restliches Wasser aus der organischen Phase entfernt. Danach wurden zu der organischen Phase 275 g Dimethylformamid zugetropft. Anschließend wurden bei 40°C 116 g Phosphoroxychlorid im Laufe von 3 Std. zugegeben und die Reaktionsmischung für 5 Stunden gerührt. Anschließend wurde auf 20°C abgekühlt und mit 160 g Methanol versetzt. Dann wurde mit ca. 180 g einer 40%igen wässrigen Natriumhydroxid-Lösung ein pH-Wert von 11 eingestellt. Anschließend wurde die Reaktionsmischung für 60 Minuten gerührt und dann das Reaktionsprodukt auf einer Nutsche isoliert und mit 160 g Methanol und 2000 g Wasser gewaschen. Das gewaschene Produkt wurde im Vakuumtrockenschrank bei einer Temperatur von 80°C und einem Druck von 200 hPa getrocknet.

Ausbeute: 141,4 g (entspricht 60% der Theorie)

### Liste der zugekauften Einsatzstoffe:

| **Bezeichnung** | **Molgewicht** | **Cas. Nr.** | **Gehalt** | **Hersteller** |
|---|---|---|---|---|
| p-Aminobenzoesäure | 137,2 | 150-13-0 | 98 | Sigma-Aldrich |
| | | | | |
| Methylisopropylketon | 86,1 | 563-80-4 | 99 | Sigma-Aldrich |
| Isopropyl methyl ketone | | | | |
| 4- Chloranilin | 127,6 | 106-47-8 | 98 | Sigma-Aldrich |
| 1,3-Indandion | 146,1 | 606-23-5 | 97 | Sigma-Aldrich |
| 5-Methylindan-1,3-dion | 160,2 | 50919-77-2 | 97 | Diverchim SA |
| 5,6 Dimethoxyindan-1,3-dion | 206,2 | 36517-91-6 | 98 | Biozol |
| 5-Methoxyindan-1,3-dion | 176,2 | 17666-95-8 | 97 | Maybridge |
| 4-Chloroindan-1,3-dion | 180,6 | 20926-88-9 | 97 | AOHChem |
| 5,6 Dichloroindan-1,3-dion | 215,0 | 93296-41-4 | 97 | Maybridge |
| 4,5,6,7 Tetrachloroindan-1.3-dion | 283,9 | 30675-13-9 | 97 | ABCR-Chemie |
| 4-Methoxy-7 methylindan-1,3-dion | 190,2 | 50919-86-3 | 98 | BOC Sciences |

Die Ergebnisse der UV/VIS Messungen und Extinktionswerte für die erfindungsgemäßen Verbindungen der Beispiele 1 bis 9 sind in Tabelle 2 aufgeführt.

**Tabelle 2**

| **Verbindung aus** | **Absorptionsmaximum UV/VIS-Spektrum¹⁾** | **E 1/1-Wert²⁾** |
|---|---|---|
| Beispiel 1 | 488 nm | 2970 |
| Beispiel 2 | 488 nm | 2880 |
| Beispiel 3 | 487 nm | 2750 |
| Beispiel 4 | 486 nm | 2780 |
| Beispiel 5 | 489 nm | 2680 |
| Beispiel 6 | 486 nm | 2950 |
| Beispiel 7 | 489 nm | 2650 |
| Beispiel 8 | 488 nm | 2710 |
| Beispiel 9 | 487 nm | 2810 |

| | | |
|---|---|---|
| ¹⁾ Die UV/VIS-Absorptionsspektren der erfindungsgemäßen Verbindungen wurden alle im Lösungsmittel 1-Methoxy-2-propylacetat (CAS-Nr. 108-65-6) gemessen. ²⁾ Der angegebene E1/1-Wert ist ein fiktiver Extinktionswert. Gemessen wird zunächst die Extinktion einer Lösung der jeweiligen Probe in 1-Methoxy-2-propylacetat in einer Küvette der Schichtdicke von 1 cm, wobei die Konzentration der Lösung so gewählt wird, dass der beobachtete Extinktionswert im Absorptionsmaximum etwa 1 beträgt. Der ermittelte Wert wird dann auf eine Konzentration von 1 Gewichtsprozent umgerechnet wodurch sich der E 1/1-Wert ergibt. | | |

### Anwendungstechnische Ergebnisse:

### A) Beschreibung der Prüfmethode "Thermostabilität"

In einem Taumelmischer wurden jeweils 2 g des zu prüfenden Farbstoffs mit 1998 g eines PA6 Granulates vom Typ Durethan B30S (Handelsprodukt der Lanxess Deutschland GmbH) mit 1% TiO2, welches 4 Stunden bei 80°C getrocknet wurde, gemischt. Dieses Gemisch wurde bei einer Massetemperatur von maximal 240°C auf einem Einschneckenextruder (Fa. Stork, 25mm Schnecke) extrudiert, mit Wasser gekühlt, mit einem Granulator der Fa. Sheer granuliert und 8 Stunden bei 80°C getrocknet. Die Hitzestabilität des erhaltenen Kunststoffgranulates wurde nach DIN EN 12877-2 ("Bestimmung der Beständigkeit der Farbe gegen Hitze beim Verarbeiten von Farbmitteln in Kunststoffen") (Methode A) auf einer Spritzgussmaschine geprüft. Als Standard wurde eine Probe bei 240°C mit einer Verweilzeit in der Schnecke von 2,5 Minuten hergestellt. Gegenüber dieser Standardprobe wurden die zu bestimmenden Proben koloristisch bewertet, die bei einer Verweilzeit von 5 Minuten und Temperaturen von 240-320°C hergestellt wurden. Proben mit einer Gesamtfarbabweichung (berechnet nach EN ISO 11664-4) von dE ≤ 3,0 wurden als stabil bei der angewandten Temperatur gewertet.

Die Ergebnisse der Bestimmung der Thermostabilität der erfindungsgemäßen Verbindungen der Beispiele 1 bis 9 sind in Tabelle 3 und die der nicht erfindungsgemäßen Verbindungen des Standes der Technik in Tabelle 4 aufgeführt.

**Tabelle 3**

| **Erfindungsgemäße Verbindung** | **Hitzestabil bis (°C)** |
|---|---|
| Beispiel 1 | 335 |
| Beispiel 2 | 340 |
| Beispiel 3 | 335 |
| Beispiel 4 | 345 |
| Beispiel 5 | 345 |
| Beispiel 6 | 335 |
| Beispiel 7 | 345 |
| Beispiel 8 | 330 |
| Beispiel 9 | 335 |

**Tabelle 4**

| **Nicht erfindungsgemäße Verbindung** | **Hitzestabil bis (°C)** |
|---|---|
| D.Y 201 (Macrolex Yellow 6G) | Entfärbung bei 240°C |
| S. Y. 93 (Macrolex Yellow 3G) | Entfärbung bei 240°C |
| S.Y 114 (Macrolex Yellow G) | 240°C |
| S.Y 160:1 (Macrolex Fluor. Yellow 10GN) | <240°C ( DE 3,6 bei 240°C ) |
| Beispiel 8 aus EP-A 3 048 138 | 320°C |
| Beispiel 9 aus EP-A 3 048 138 | 320°C |

## Patentansprüche

1. Farbstoff der Formel (I) 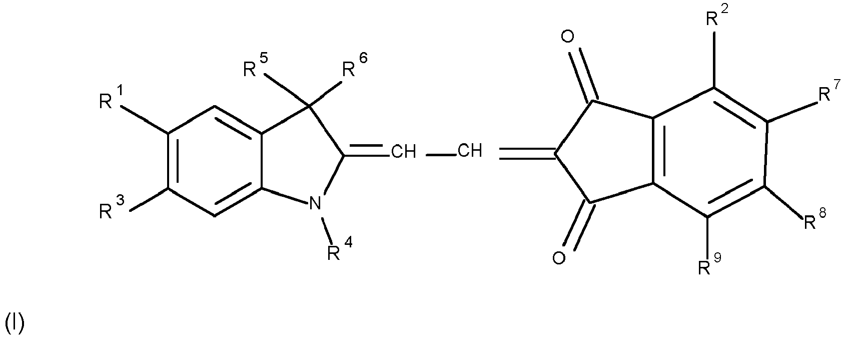 worin
R¹ für Wasserstoff, Halogen, COOH oder COOR¹⁰ steht,
R² für Wasserstoff, Halogen, C₁-C₄-Alkyl das gegebenenfalls ein- bis dreifach, gleich oder verschieden durch Halogen substituiert ist, oder für C₁-C₄-Alkoxy steht,
R³ für Wasserstoff, Halogen, COOH, COOR¹¹ oder CN steht,
R⁴ für C₁-C₄- Alkyl oder Phenyl steht,
R⁵ und R⁶ jeweils unabhängig voneinander für C₁-C₄-Alkyl stehen,
R⁷ für Wasserstoff, Halogen, geradkettiges oder verzweigtes C₁-C₄-Alkyl das gegebenenfalls ein- bis dreifach, gleich oder verschieden durch Halogen substituiert ist, oder für C₁-C₄-Alkoxy steht,
R⁸ für Wasserstoff, Halogen, C₁-C₄-Alkyl das gegebenenfalls ein- bis dreifach, gleich oder verschieden durch Halogen substituiert ist oder für C₁-C₄-Alkoxy steht,
R⁹ für Wasserstoff, Halogen, C₁-C₄-Alkyl das gegebenenfalls ein- bis dreifach, gleich oder verschieden durch Halogen substituiert ist, oder für C₁-C₄-Alkoxy steht und
R¹⁰ und R¹¹ jeweils unabhängig voneinander für C₁-C₄-Alkyl stehen,
mit der Bedingung, dass R¹ und R³ nicht gleichzeitig für Wasserstoff stehen.

2. Farbstoff gemäß Anspruch 1, **dadurch gekennzeichnet, dass** in Formel (I)
R¹ für Wasserstoff, Fluor, Chlor, COOH oder COOR¹⁰ steht,
R² für Wasserstoff, Fluor, Chlor, Methyl, Ethyl, Trifluormethyl, Methoxy oder Ethoxy steht,
R³ für Wasserstoff, Fluor, Chlor, COOH, COOR¹¹ oder CN steht,
R⁴ für Methyl, Ethyl oder Phenyl steht,
R⁵ und R⁶ jeweils unabhängig voneinander für Methyl oder Ethyl stehen,
R⁷ für Wasserstoff, Fluor, Chlor, Methyl, Ethyl, Trifluormethyl, Methoxy oder Ethoxy steht,
R⁸ für Wasserstoff, Fluor, Chlor, Methyl, Ethyl, Trifluormethyl, Methoxy oder Ethoxy steht,
R⁹ für Wasserstoff, Fluor, Chlor, Methyl, Ethyl, Trifluormethyl, Methoxy oder Ethoxy steht und
R¹⁰ und R¹¹ jeweils unabhängig voneinander für Methyl oder Ethyl stehen,
mit der Bedingung, dass R¹ und R³ nicht gleichzeitig für Wasserstoff stehen.

3. Farbstoff gemäß Anspruch 1 oder 2, **dadurch gekennzeichnet, dass** in Formel (I)
R¹ für Chlor oder COOR¹⁰ steht,
R² für Wasserstoff, Fluor, Chlor, CF₃, Methyl oder Methoxy steht,
R³ für Wasserstoff steht,
R⁴ für Methyl steht
R⁵ und R⁶ für Methyl stehen
R⁷ für Wasserstoff, Fluor, Chlor, Methyl oder Methoxy steht,
R⁸ für Wasserstoff, Fluor, Chlor, Methyl oder Methoxy steht,
R⁹ für Wasserstoff, Fluor, Chlor, Methyl oder Methoxy steht und
R¹⁰ für Methyl steht.

4. Verwendung von mindestens einem Farbstoff gemäß wenigstens einem der Ansprüche 1 bis 3 zum Massefärben von Kunststoffen.

5. Verwendung gemäß Anspruch 4, **dadurch gekennzeichnet, dass** es sich um mindestens einen Kunststoff aus der Reihe der Vinylpolymere, Polyester, Polyolefine, Polycarbonate und Polyamide handelt.

6. Verwendung gemäß wenigstens einem der Ansprüche 4 oder 5, **dadurch gekennzeichnet, dass** es sich bei dem Kunststoff um Polyamid 6 und/oder Polyamid 6.6 handelt.

7. Verwendung gemäß wenigstens einem der Ansprüche 4 bis 6, **dadurch gekennzeichnet, dass** der Farbstoff in einer Menge von 0,0001 bis 1 Gewichtsprozent, insbesondere 0,01 bis 0,5 Gewichtsprozent, bezogen auf die Menge an Kunststoff eingesetzt wird.

8. Verfahren zum Massefärben von Kunststoffen, **dadurch gekennzeichnet, dass** mindestens ein Farbstoff gemäß wenigstens einem der Ansprüche 1 bis 3 mit mindestens einem Kunststoff, vorzugsweise in Granulatform, trocken vermischt oder vermahlen wird und dieses Gemisch geschmolzen und homogenisiert wird.

9. Verfahren zum Massefärben von Kunststoffen, **dadurch gekennzeichnet, dass** mindestens ein Farbstoff gemäß wenigstens einem der Ansprüche 1 bis 3 zu einer geschmolzenen, mindestens einen Kunststoff enthaltenden Kunststoffmasse zugegeben und diese dann homogenisiert wird.

10. Verfahren zum Massefärben von Kunststoffen, **dadurch gekennzeichnet, dass** mindestens ein Farbstoff gemäß wenigstens einem der Ansprüche 1 bis 3 mit den monomeren Ausgangskomponenten zur Herstellung mindestens eines Kunststoffes vermischt wird und die Mischung anschließend polymerisiert wird.

11. Verfahren zum Massefärben von Polymethylmethacrylat (PMMA) **dadurch gekennzeichnet, dass** mindestens ein Farbstoff gemäß wenigstens einem der Ansprüche 1 bis 3 mit mindestens einem Methacrylsäure-methylestermonomer vermischt oder darin gelöst wird und diese Mischung oder Lösung dann in Gegenwart mindestens eines Polymerisationskatalysators polymerisiert wird.

12. Kunststoffzusammensetzung, insbesondere Polyamidzusammensetzung oder Polymethylmethacrylat, **dadurch gekennzeichnet, dass** sie mindestens einen Farbstoff gemäß wenigstens einem der Ansprüche 1 bis 3 enthält.

13. Formteile, **dadurch gekennzeichnet, dass** sie mindestens eine Kunststoff-Zusammensetzung gemäß Anspruch 12 enthalten.

14. Verfahren zur Herstellung eines Farbstoffs gemäß Anspruch 1, **dadurch gekennzeichnet, dass** mindestens ein Aldehyd der Formel (II) 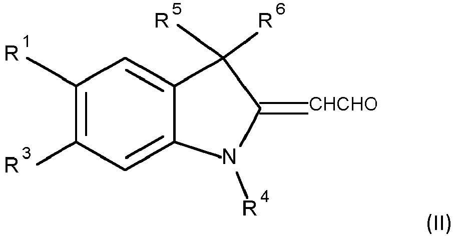 worin
R¹, R³, R⁴, R⁵ und R⁶ die in Anspruch 1 angegebenen Bedeutungen haben,
mit mindestens einem Indan-1,3-dion Derivat der Formel (III) 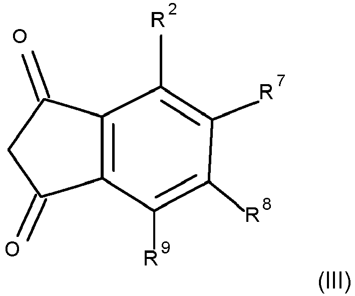
worin
R² und R⁷ die in Anspruch 1 angegebenen Bedeutungen haben, umsetzt.

## Claims

1. Dye of the formula (I) 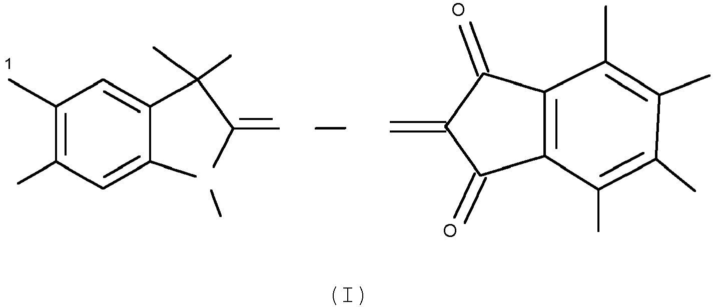 wherein
R¹ is hydrogen, halogen, COOH or COOR¹⁰,
R² is hydrogen, halogen, C₁-C₄-alkyl which is optionally mono- to trisubstituted, identically or differently, by halogen, or is C₁-C₄-alkoxy,
R³ is hydrogen, halogen, COOH, COOR¹¹ or CN,
R⁴ is C₁-C₄-alkyl or phenyl,
R⁵ and R⁶ are each independently C₁-C₄-alkyl,
R⁷ is hydrogen, halogen, straight-chain or branched C₁-C₄-alkyl which is optionally mono- to trisubstituted, identically or differently, by halogen, or is C₁-C₄-alkoxy,
R⁸ is hydrogen, halogen, C₁-C₄-alkyl which is optionally mono- to trisubstituted, identically or differently, by halogen, or is C₁-C₄-alkoxy,
R⁹ is hydrogen, halogen, C₁-C₄-alkyl which is optionally mono- to trisubstituted, identically or differently, by halogen, or is C₁-C₄-alkoxy
R¹⁰ and R¹¹ are each independently C₁-C₄-alkyl,
with the condition that R¹ and R³ are not both hydrogen.

2. Dye according to Claim 1, **characterized in that**, in formula (I)
R¹ is hydrogen, fluorine, chlorine, COOH or COOR¹⁰,
R² is hydrogen, fluorine, chlorine, methyl, ethyl, trifluoromethyl, methoxy or ethoxy,
R³ is hydrogen, fluorine, chlorine, COOH, COOR¹¹ or CN,
R⁴ is methyl, ethyl or phenyl
R⁵ and R⁶ are each independently methyl or ethyl,
R⁷ is hydrogen, fluorine, chlorine, methyl, ethyl, trifluoromethyl, methoxy or ethoxy,
R⁸ is hydrogen, fluorine, chlorine, methyl, ethyl, trifluoromethyl, methoxy or ethoxy,
R⁹ is hydrogen, fluorine, chlorine, methyl, ethyl, trifluoromethyl, methoxy or ethoxy and
R¹⁰ and R¹¹ are each independently methyl or ethyl,
with the condition that R¹ and R³ are not both hydrogen.

3. Dye according to Claim 1 or 2, **characterized in that**, in formula (I)
R¹ is chlorine or COOR¹⁰,
R² is hydrogen, fluorine, chlorine, CF₃, methyl or methoxy,
R³ is hydrogen,
R⁴ is methyl,
R⁵ and R⁶ are methyl,
R⁷ is hydrogen, fluorine, chlorine, methyl or methoxy,
R⁸ is hydrogen, fluorine, chlorine, methyl or methoxy,
R⁹ is hydrogen, fluorine, chlorine, methyl or methoxy and
R¹⁰ is methyl.

4. Use of at least one dye according to at least one of Claims 1 to 3 for the bulk colouration of plastics.

5. Use according to Claim 4, **characterized in that** said plastic is at least one plastic from the series of vinyl polymers, polyesters, polyolefins, polycarbonates and polyamides.

6. Use according to at least one of Claims 4 or 5, **characterized in that** the plastic is polyamide 6 and/or polyamide 6.6.

7. Use according to at least one of Claims 4 to 6, **characterized in that** the dye is used in an amount from 0.0001 to 1 percent by weight, especially 0.01 to 0.5 percent by weight, based on the amount of plastic.

8. Method for the bulk colouration of plastics, **characterized in that** at least one dye according to at least one of Claims 1 to 3 is mixed in dry form or is ground with at least one plastic, preferably in the form of granules, and this mixture is melted and homogenized.

9. Method for the bulk colouration of plastics, **characterized in that** at least one dye according to at least one of Claims 1 to 3 is added to a molten plastic material comprising at least one plastic and this is then homogenized.

10. Method for the bulk colouration of plastics, **characterized in that** at least one dye according to at least one of Claims 1 to 3 is mixed with the monomeric starting components for producing at least one plastic and the mixture is subsequently polymerized.

11. Method for the bulk colouration of polymethyl methacrylate (PMMA), **characterized in that** at least one dye according to at least one of Claims 1 to 3 is mixed with at least one methyl methacrylate monomer or is dissolved therein and this mixture or solution is then polymerized in the presence of at least one polymerization catalyst.

12. Plastic composition, especially a polyamide composition or polymethyl methacrylate, **characterized in that** said composition comprises at least one dye according to at least one of Claims 1 to 3.

13. Mouldings, **characterized in that** said mouldings comprise at least one plastic composition according to Claim 12.

14. Method for producing a dye according to Claim 1, **characterized in that** at least one aldehyde of the formula (II) 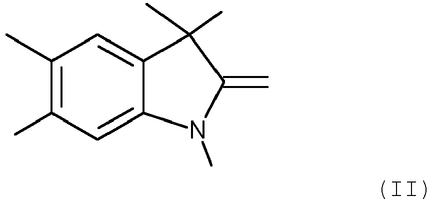 wherein
R¹, R³, R⁴, R⁵ and R⁶ have the definitions specified in Claim 1,
is reacted with at least one indane-1,3-dione derivative of the formula (III) 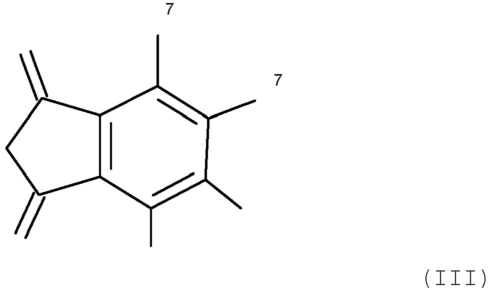
wherein
R² and R⁷ have the definitions specified in Claim 1.

## Revendications

1. Colorant de formule (I) 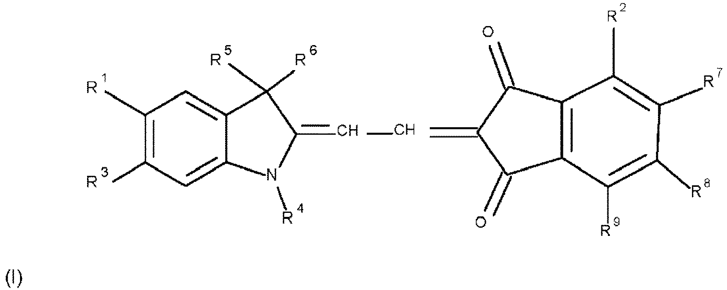 dans laquelle
R¹ représente hydrogène, halogène, COOH ou COOR¹⁰,
R² représente hydrogène, halogène, C₁-C₄-alkyle qui est le cas échéant monosubstitué à trisubstitué, de manière identique ou différente par halogène, ou représente C₁-C₄-alcoxy,
R³ représente hydrogène, halogène, COOH, COOR¹¹ ou CN,
R⁴ représente C₁-C₄-alkyle ou phényle,
R⁵ et R⁶ représentent à chaque fois, indépendamment l'un de l'autre, C₁-C₄-alkyle ;
R⁷ représente hydrogène, halogène, C₁-C₄-alkyle linéaire ou ramifié, qui est le cas échéant monosubstitué à trisubstitué, de manière identique ou différente par halogène, ou représente C₁-C₄-alcoxy,
R⁸ représente hydrogène, halogène, C₁-C₄-alkyle qui est le cas échéant monosubstitué à trisubstitué, de manière identique ou différente par halogène, ou représente C₁-C₄-alcoxy,
R⁹ représente hydrogène, halogène, C₁-C₄-alkyle qui est le cas échéant monosubstitué à trisubstitué, de manière identique ou différente par halogène, ou représente C₁-C₄-alcoxy et
R¹⁰ et R¹¹ représentent à chaque fois, indépendamment l'un de l'autre, C₁-C₄-alkyle ;
à condition que R¹ et R³ ne représentent pas simultanément hydrogène.

2. Colorant selon la revendication 1, **caractérisé en ce que**, dans la formule (I),
R¹ représente hydrogène, fluor, chlore, COOH ou COOR¹⁰,
R² représente hydrogène, fluor, chlore, méthyle, éthyle, trifluorométhyle, méthoxy ou éthoxy,
R³ représente hydrogène, fluor, chlore, COOH, COOR¹¹ ou CN,
R⁴ représente méthyle, éthyle ou phényle,
R⁵ et R⁶ représentent, à chaque fois indépendamment l'un de l'autre, méthyle ou éthyle,
R⁷ représente hydrogène, fluor, chlore, méthyle, éthyle, trifluorométhyle, méthoxy ou éthoxy,
R⁸ représente hydrogène, fluor, chlore, méthyle, éthyle, trifluorométhyle, méthoxy ou éthoxy,
R⁹ représente hydrogène, fluor, chlore, méthyle, éthyle, trifluorométhyle, méthoxy ou éthoxy et
R¹⁰ et R¹¹ représentent, à chaque fois indépendamment l'un de l'autre, méthyle ou éthyle,
à condition que R¹ et R³ ne représentent pas simultanément hydrogène.

3. Colorant selon la revendication 1 ou 2, **caractérisé en ce que**, dans la formule (I),
R¹ représente chlore ou COOR¹⁰,
R² représente hydrogène, fluor, chlore, CF₃, méthyle ou méthoxy,
R³ représente hydrogène,
R⁴ représente méthyle,
R⁵ et R⁶ représentent méthyle,
R⁷ représente hydrogène, fluor, chlore, méthyle ou méthoxy,
R⁸ représente hydrogène, fluor, chlore, méthyle ou méthoxy,
R⁹ représente hydrogène, fluor, chlore, méthyle ou méthoxy et
R¹⁰ représente méthyle.

4. Utilisation d'au moins un colorant selon au moins l'une quelconque des revendications 1 à 3 pour la coloration dans la masse de matériaux synthétiques.

5. Utilisation selon la revendication 4, **caractérisée en ce qu'**il s'agit d'au moins un matériau synthétique de la série des polymères de vinyle, des polyesters, des polyoléfines, des polycarbonates et des polyamides.

6. Utilisation selon au moins l'une quelconque des revendications 4 ou 5, **caractérisée en ce qu'**il s'agit, pour le matériau synthétique, de polyamide 6 et/ou de polyamide 6.6.

7. Utilisation selon au moins l'une quelconque des revendications 4 à 6, **caractérisée en ce que** le colorant est utilisé en une quantité de 0,0001 à 1% en poids, en particulier de 0,01 à 0,5% en poids, par rapport à la quantité de matériau synthétique.

8. Procédé pour la coloration dans la masse de matériaux synthétiques, **caractérisé en ce qu'**au moins un colorant selon au moins l'une quelconque des revendications 1 à 3 est mélangé ou broyé à sec avec au moins un matériau synthétique, de préférence sous forme de granulat et ce mélange est fondu et homogénéisé.

9. Procédé pour la coloration dans la masse de matériaux synthétiques, **caractérisé en ce qu'**au moins un colorant selon au moins l'une quelconque des revendications 1 à 3 est ajouté à une masse de matériau synthétique fondue, contenant au moins un matériau synthétique et cette masse est ensuite homogénéisée.

10. Procédé pour la coloration dans la masse de matériaux synthétiques, **caractérisé en ce qu'**au moins un colorant selon au moins l'une quelconque des revendications 1 à 3 est mélangé avec les composants de départ monomères pour la fabrication d'au moins un matériau synthétique et le mélange est ensuite polymérisé.

11. Procédé pour la coloration dans la masse de poly(méthacrylate de méthyle) (PMMA), **caractérisé en ce qu'**au moins un colorant selon au moins l'une quelconque des revendications 1 à 3 est mélangé avec au moins un monomère d'ester méthylique d'acide méthacrylique ou dissous dans celui-ci et le mélange ou la solution est ensuite polymérisé(e) en présence d'au moins un catalyseur de polymérisation.

12. Composition de matériau synthétique, en particulier composition de polyamide ou de poly(méthacrylate de méthyle), **caractérisée en ce qu'**elle contient au moins un colorant selon au moins l'une quelconque des revendications 1 à 3.

13. Pièces façonnées, **caractérisées en ce qu'**elles contiennent au moins une composition de matériau synthétique selon la revendication 12.

14. Procédé pour la préparation d'un colorant selon la revendication 1, **caractérisé en ce qu'**au moins un aldéhyde de formule (II) 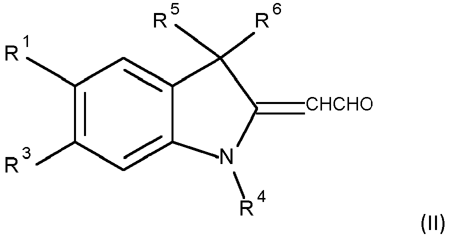 dans laquelle
R¹, R³, R⁴, R⁵ et R⁶ présentent les significations indiquées dans la revendication 1,
est transformé avec au moins un dérivé d'indane-1,3-dione de formule (III) 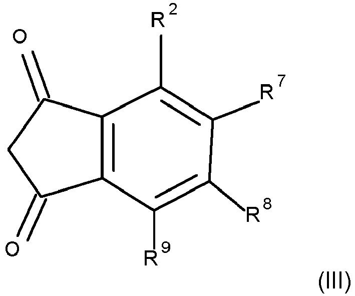
dans laquelle
R² et R⁷ présentent les significations indiquées dans la revendication 1.
